# EUROPEAN PATENT APPLICATION

(11) **EP 4 378 313 A1**
(43) Date of publication of application: **05.06.2024**
(21) Application number: 22919274.5
(22) Date of filing: 21.10.2022
(51) Int. Cl.: A01N 63/20, A01N 63/23, A01N 65/28, A01N 65/44

(54) **AGRICULTURAL BIOINSECTICIDE COMPOSITION CONTAINING BREVIBACILLUS LATEROSPORUS, DIFFERENT SUBSPECIES OF BACILLUS THURINGIENSIS AND BOTANICAL EXTRACTS OF CITRONELLA AND MELALEUCA, INDUSTRIAL PROCESS AND USE OF SAME ON CROPS OF AGRICULTURAL IMPORTANCE**

(71) Applicant: Total Biotecnologia Indústria e Comércio S.A., 81460-020 Curitiba - PR (BR)
(72) Inventor: FUKAMI, Josiane, 81460-020 Curitiba, Paraná (BR); GOMES, Douglas Fabiano, 81460-020 Curitiba, Paraná (BR); MARCOLINO GOMES, Juliana, 81460-020 Curitiba, Paraná (BR); DE ASSIS FILHO, Jonas Hipólito, 81460-020 Curitiba, Paraná (BR)
(74) Representative: Winter, Brandl - Partnerschaft mbB
(86) International application number: PCT/BR2022/050407
(87) International publication number: WO 2024/082036

(57) **Abstract**

The present invention is related to a bioinsecticide agricultural composition, as well as the industrial process, and the use of one or more of the species of the genre *Brevibacillus laterosporus* and subspecies of *Bacillus thuringiensis,* apart from the association of botanical extracts to increase the insecticide efficiency of the microorganisms applied to the field in plants of agricultural interest. Surprisingly, the combination of the three microorganisms with the addition of botanical extract containing tea tree essential oil and/or citronella essential oil potentialize the bioinsecticide effect against insects of the Lepidoptera order, anticipating the control, as well as acting on several cultures with agronomic interest, such as corn, wheat, cotton.

## Description

### FIELD OF THE INVENTION

The present invention is related to an agricultural composition with bioinsecticide action, containing one or more subspecies of the genre *Bacillus thuringiensis* and *Brevibacillus laterosporus* in association with botanical extracts, including the industrial process for obtaining the composition and the use thereof in cultures with agricultural importance with superior performance in the control of insects of the Lepidoptera order, reducing losses caused by these pests, minimizing the use of chemical agricultural pesticides and, therefore, contributing to the agricultural sustainability.

### DESCRIPTION OF RELATED ART

During the past years, the excessive and successive use of insecticide molecules with chemical synthesis with similar action mechanisms and further, with the advent of the genetically modified plants, particularly corn Bt resistant to armyworm such as the *Spodoptera frugiperda* (fall armyworm), which comprises in their genome genes of the Cry protein of the microorganism *Bacillus thuringiensis,* have caused undesirable selections in the populations of pest-insects with agricultural importance. The sum of these practices promotes the increase of the resistance of these insect populations to the traditional solutions (pesticides), resulting in the reduction of the effectiveness of the technologies currently used. The final consequence is the increase of the dosages and frequency of application of the chemical pesticides in the crops. In this manner, the adoption of pesticides becomes increasingly frequent and voluminous, going against a more healthy, rational, and sustainable agriculture.

For this reason, added to the concern as to the quality of the foods, studies have been intensified about new control tools and handling of pest-insects (Tavares et al., 2009). Among the alternative practices there is emphasis on the biological control that employs microorganisms, such as fungi and bacteria, whereby this is an important tool for handling the resistance and the conscious use of pesticides.

The bacteria of the genre Bacillus are well-known, particularly *B. thuringiensis,* which, since 1915, were characterized as having potential insecticide action against insects of the Lepidoptera, Diptera and Coleoptera orders (Habbib and Andrade, 1986; Glare and O'Callagham, 2000). In 1938 there were developed the first products based on this approach for biological control with the commercialization of this technology in France (Daust, 1990), and, next, they were spread all over the world. The subspecies of *B.thuringiensis* have entomopathogenic activity, since during the sporulation phase there are produced, among other insecticide toxins, the Cry proteins, being encoded by the *cry* genes (Glare and O'Callagham, 2000). The different subspecies of *B. thuringiensis* present, in their respective genomes, information for encoding distinct types of Cry proteins, whereby these differences are reflected in the specificity and efficiency of the action on insect control. Currently there exist 93 subspecies described (Genbank,2021). However, the most studied subspecies are, *B. thuringienesis* subsp. kurstaki; *B. thuringienesis* subsp. *aizawai*; *B. thuringienesis* subsp. *israelenses*; *B. thuringienesis* subsp. *tenebrionis*; *B. thuringienesis* subsp. *galleriae*; *B. thuringienesis* subsp. *pakistani*; *B. thuringienesis* subsp. *tochigiensis,* among others.

Only in Brazil there exist more than 30 registrations with products containing only one subspecies of *Bacillus thuringiensis.* Since then, no other microbial genre was found that would make up this group with strong bioinsecticide action against the order of the Lepidoptera. Due to the limited variety of action mechanisms, even when referring to biological products, the efficiency in the control of the technology that employs only one controller microorganism is not sufficiently effective in the control of Lepidoptera, whereby the development of new tools is necessary, which make use of different action mechanisms in a single product.

*Brevibacillus laterosporus* is Gram positive bacteria, aerobic with endospore formation (resistance structures), which are characterized by the ability to produce a parasporal lamelar inclusion in the form of a canoe adjacent the spore. Several studies report the action mechanisms, demonstrating that this species is able to produce enzymes which act as fungicide, antibacterial, insecticides, and mainly used as effective prebiotics (Afrikian, 1989; Orlova et al.,1998; Ghazanchyan et al., 2018). The insecticide action of this species against Coleoptera larvae and velvetbean caterpillar *Anticarsia gemmatalis* (Oliveira et al., 2004) has already been described. However, these same authors did not find any effect for the fall armyworm *Spodoptera frugiperda.*

In addition to the selection of new microorganisms for an agricultural composition, the identification and isolation of vegetable compounds having insecticide properties can also be a promising alternative in the development of new products in handling pest insects, generating a new tool relative to the use of synthetic agrochemicals, since there are reports of the increase in resistance of pests in response to these agrochemical substances (Roush and Daly 1990; Raveau et al. 2020).

In a review, carried out by Paredes-Sánchez and collaborators (2021), there were characterized several compounds extracted from plants with insecticide action against the fall armyworm *Spodoptera frugiperda,* among them being: *neem* oil(*Azadirachta indicae*); extract from Atlantic forest fruit tree (*Duguetia lanceolata*); extract from a weed (*Polygonum hydropiperoides*); extract from fruit tree such as the papaya (Carica papaya), orange (*Citrus sinensis*), citronella lemon (*C. limonia*), lemon (*Citrus limon*); among others. Another study containing basil essential oil (*Ocimum basilicum* L.), which active principle the linalool (Silva et al., 2017) presented high insecticide action against *Spodoptera frugiperda.* Although there are several reports of botanical extracts against caterpillars, there are few reports using the action of the citronella essential oil (*Cymbopogon winterianus and Cymbopogon nardus*) and tea tree essential oil (*Melaleuca alternifólia*) with bioinsecticide function, and further, combined with microorganisms in a single product, compatible and effective for agricultural application.

Industrial production, see detailed technical description, of the biological insecticide comprised by *Brevibacillus laterosporos, Bacillus thuringiensis* subsp. *kurstaki* and *Bacillus thuringiensis* subsp. *aizawai* undergoes a complex process which guarantees the high cellular concentration during the exponential growth phase of bacteria, and, followed by the induction of the spore formation, the parasporal body and the crystals industrially induced by *Brevibacillus laterosporos* and *Bacillus thuringiensis,* respectively. This process involves specific parameters such as growth temperature, air volume, agitation, and pressure (related to the rate of oxygen dissolved in the culture medium). Thus, the industrial production with fundamental fermentative parameters for providing the species of *Brevibacillus* and *Bacillus* which guarantee the stability of the parasporal body and crystals with high cell viability, enabling the combination with citronella and/or tea tree botanical extracts in a single product for application as an agricultural bioinsecticide composition becomes an important tool for handling and biological control of pest-insects with agronomic interest.

In addition to the control, the diversity of action mechanisms, combined for the first time in an agricultural composition, involving different species of bacteria and botanical extracts will contribute strongly to the reduction of the selection of populations that are resistant to agricultural pests and, consequently, will reduce the need for frequent and voluminous application of chemical pesticides, promoting greater sustainability to the agribusiness.

The new insecticide composition is able to reduce the selection pressure, extending the effects thereof to the longevity of the genetic improvement technologies (conventional or transgenic) allowing the optimization of investments in new materials for agriculture.

The commercial products containing Bacillus thuringiensis undergo processes with energetic and economic costs such as the tangential filtering or drying with spray driers. Said production cost reduces the ability of the biotechnological industry to compete in the market of pesticides with chemical synthesis and low cost.

The current state of the art does not allow the control of insects with *Bacillus thuringiensis* without additional processes to the steps of fermentation and formulation. The new composition and the industrial process thereof innovate by waiving said steps, increasing competitiveness of the new solutions for sustainable agriculture.

The efficiency of the *Brevibacillus* sp. as insecticide for the most problematic caterpillar of the tropical agriculture (*Spodoptera frugiperda*) is not, according to the current state of the art, sufficient to replace pesticides and generate commercial products in industrial scale.

The mixture of vegetable extracts and insecticide microorganisms is not viable in industrial scale as per the current state of the art.

### REFERENCES

Afrikian, E.G. Bacterial insecticides, in: Bioprocess Engineering. The First Generation, Delhi, India, Elsevier Co, L., p. 276-290, 1989.
Genbank. 2021. Available at: <http://www.ncbi.nlm.nih.gov/genbank/>. Access on Nov 30, 2021.
Ghazanchyan, N.L.; Kinosyan, M.H.; Tadevosyan, P.E.; Khachaturyan, N.S.; Afrikian, E.G. BREVIBACILLUS LATEROSPORUS as perspective source of new bioinsecticides, Annals of Agrarian Sciences, 2018.
Daoust, R. A. Commercialization of bacterial insecticides. In: Proceedings and abstracts, Vth International Colloquium on Invertebrate Pathology and Microbial Control, Adelaide, Australia, 20-24 August 1990. Department of Entomology, University of Adelaide, p. 7-11, 1990.
GLARE, T. R.; O' CALLAGHAN, M. 2000. Bacillus thuringiensis: biology, ecology, and safety. Chichester: John Wiley, 350p.
Habib, M. E. M.; Andrade, C. F. S. "Entomopathogenic bacteria". In: Microbial Control of Insects. Alves, SB (Coord.).", 1986.
De Oliveira, E. J., Rabinovitch, L., Monnerat, R. G., Passos, L. K. J., & Zahner, V. (2004). Molecular characterization of Brevibacillus laterosporus and its potential use in biological control. Applied and environmental microbiology, 70(11), 6657-6664.
Orlova, M. V.; Smirnova, T. A.; Ganushkina, L. A.; Yacubovich, V. Y.; Azizbekyan, R. R. Insecticidal Activity of Bacillus laterosporus, Appl. Environ. Microbiol. v.64, p.2723- 2725, 1998.
Paredes-Sánchez, F. A.; Rivera, G.; Bocanegra-Garcia, V.; Martínez-Padrón, H. Y.; Berrones-Morales, M.; Niño-García, N.; Herrera-Mayorga, V. Advances in Control Strategies against Spodoptera frugiperda. A Review. Molecules, v. 26, p.5587, 2021.
Raveau R, Fontaine J, Sahraoui ALH (2020) Essential oils as potential alternative biocontrol products against plant pathogens and weeds: a review. Foods 9:365.
Roush RT, Daly JC (1990) The role of population genetics in resistance research and management. In Roush R, Tabashnik BE (eds) Pesticide Resistance in Arthropods, Springer US, New York.
Silva, S. M.; Cunha, J. P. A. R. D.; Carvalho, S. M. D.; Zandonadi, C. H. S.; Martins, R. C.; Chang, R. Ocimum basilicum essential oil combined with deltamethrin to improve the management of Spodoptera frugiperda. Ciência e Agrotecnologia, v.41, p.665-675,2017.
Tavares, W. S; Cruz, I.; Petacci, F.; de Assis Júnior, S. L.; de Sousa Freitas, S.; Zanuncio, J. C.; Serrão, J. E Potential use of Asteraceae extracts to control Spodoptera frugiperda (Lepidoptera: Noctuidae) and selectivity to their parasitoids Trichogramma pretiosum (Hymenoptera: Trichogrammatidae) and Telenomus remus (Hymenoptera: Scelionidae). Industrial Crops Products, v.31, p.384-388, 2009.

### SUMMARY OF THE INVENTION

The present invention consists of an agricultural composition that employes microorganisms of the species *Brevibacillus laterosporus* and *Bacillus thuringiensis,* wherein the concentration of each microbiological active comprises the range of 1,0×10⁷ - 1,0×10¹⁰ viable spores/mL, associated with botanical extracts, preferably tea tree and citronella essential oils in the range between 0,01 - 5,0 % (v/v), able to potentialize the bioinsecticide effect against pests with agricultural interest in a technical solution that involves the industrial process and use of same. The applicability of the invention is provided, mainly, for application against pests with agricultural interest, whereby it can be used in different cultures and product concentrations, as well as different stages of the phenological stage of plants. The applications are carried out, preferably, via foliar spraying, in which manner the composition is able to reach the targets. Additionally, the applications can vary regarding dosage and frequency (number of applications).

The present invention teaches that, surprisingly, it is possible to develop a biotechnological solution (in industrial scale) containing one or more strains of *Brevibacillus laterosporus* and *Bacillus thuringiensis,* associated with botanical extracts that are able to potentialize the bioinsecticide effects against pests with agricultural importance.

Advantageously, the present invention allows obtaining an agricultural composition which can be applied to the field for handling the resistance of agrochemicals and transgenic cultivars with Bt technology in several cultures with agronomic interest, such as corn, soybean, cotton, among others.

As will be understood by a person skilled in the art, the present invention provides additional parameters for the method of producing an agricultural composition formed by two or more subspecies of *Brevibacillus laterosporus* and *Bacillus thuringiensis* fermented in industrial scale, demonstrating the necessary parameters for inducing the formation of endospores and insecticide toxins such as the Cry proteins (crystals) and parasporal bodies in the shape of canoe, such as parameters for pressure, temperature, oxygenation (air volume and agitation) and composition of the culture media, enabling obtaining a biotechnological product with very superior performance to that of the ones currently available.

In a first embodiment, the present invention provides a process for producing an agricultural composition comprising the steps of:
(a) fermentation of the microorganisms comprising three species, namely *Bacillus thuringiensis* subsp. *kurstaki, Bacillus thuringiensis* subsp. *aizawai and Brevebacillus laterosporus* for obtaining an agricultural composition with induction of endospores and crystals and insecticide parasporal toxins able to potentialize the bioinsecticide effect over targets with agronomic interest, through the specific formulation for each microorganism during the industrial process; and
(b) formulation of a biotechnological product comprised by the mixture of bacteria, associated with the tea tree essential oil botanical extract and/or citronella essential oil in a technical solution that allows the application in agriculture as bioinsecticide.

In a surprising manner, the present invention has as its preferred embodiment the potentializing of the bioinsecticide effect in cultures with agronomic interest for high levels of control and industrial viability.

In a surprising manner, the present invention has as its preferred embodiment the potentializing of the bioinsecticide effect with the mixture of microorganisms of *Brevibacillus laterosporus* and subspecies of *Bacillus thuringiensis* able to control targets that are difficult to handle, such as the fall armyworm (*Spodoptera frugiperda*).

### BRIEF DESCRIPTION OF THE FIGURES

For a more complete understanding of the invention, reference must be made now to the embodiments of the invention illustrated in more detail in the attached figures and described by means of the embodiments of the invention.

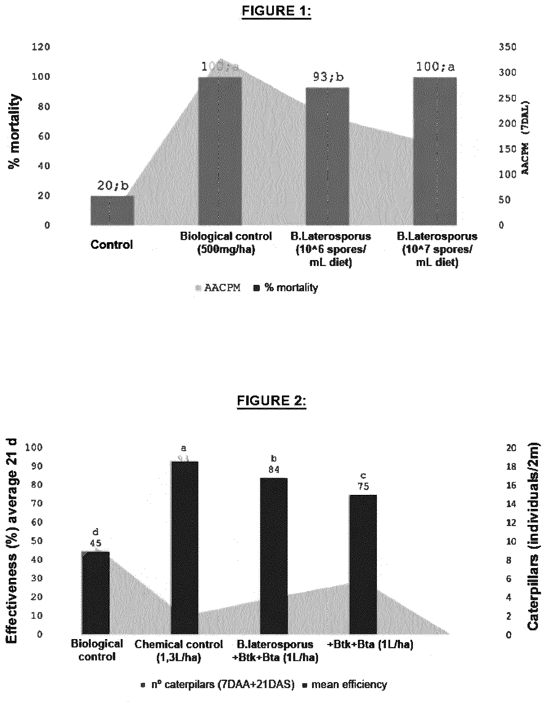

Figure 1 illustrates the mortality percentage of the fall armyworm *Spodoptera frugiperda* 7 Days after Release (7 DAR) in diet inoculated with different concentrations of *Brevibacillus laterosporus* and the Area Under the Mortality Progression Curve (AUMPC). * Results of 8 treatments and 5 repetitions; data not presented since they are not relevant to the study. Means followed by the same letters do not differ from each other by the SK test at 5% significance.
Figure 2 illustrates the efficiency percentage of the fall armyworm *Spodoptera frugiperda* 21 Days After the second application in the wheat culture * Results of 4 treatments and 4 repetitions with Randomized Block Design (DIC) . Means followed by the same letters do not differ from each other by the SK test at 5% significance.

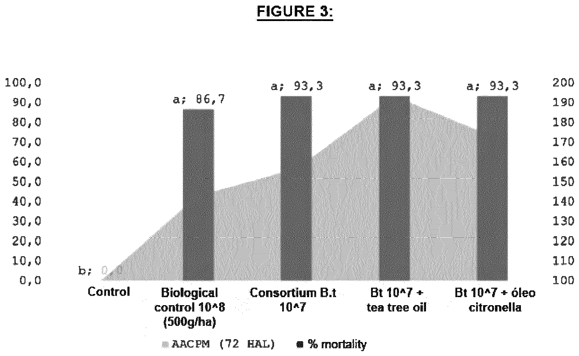

Figure 3 illustrates the mortality percentage of the fall armyworm *Spodoptera frugiperda* 72 Hours After Release (HAL) in bio-trial with application of the consortium of subspecies of *Bacillus thuringiensis* associated with tea tree essential oil or citronella essential oil and the Area Under the Mortality Progression Curve (AUMPC). * Results of 9 treatments and 5 repetitions, data not presented since they are not relevant to the study. Means followed by the same letters do not differ from each other by the SK test at 5% significance.

### DETAILED DESCRIPTION OF THE INVENTION

In a preferred embodiment, according to the present invention, the fermentation step (a)) of the species of *Brevibacillus laterosporus* and subspecies of *Bacillus thuringiensis* per batch occurs for approximately 24 - 168 hours.

In a preferred embodiment, the method of the present invention comprises the sequential expansion (scaling) of the culture of *Brevibacillus laterosporus* and subspecies of *Bacillus thuringiensis* for inoculation of the fermentation culture. Preferably, the sequential expansion starts at volumes of 100 L, which serves as inoculum for 1 L. This, in turn, is inoculated in 10 L, which are then inoculated in two flasks in 180 L tanks, are transferred to reactors containing 2.000 L, and which, finally, are transferred to 6.000 L reactors.

In a preferred embodiment, the species of *Brevibacillus* and subspecies of *Bacillus thuringiensis* are expanded in flasks of 100 mL by incubation in orbital agitator from 80 rpm to 200 rpm. The incubation time is preferably from 8 hours to 48 hours. Preferably, the species of *Brevibacillus* and subspecies of *Bacillus thuringiensis* are cultivated in flasks with about 1 L of culture medium by incubation in orbital agitator at 80 rpm to 200 rpm.

In a preferred embodiment, the air flow of the stainless steel flasks containing 10 L for the cultivation containing the species of *Brevibacillus* and subspecies of *Bacillus thuringiensis* is of 0,25 to 1,5 Nm³/h (= 0,41 - 2,5 vvm), and the incubation time if preferably about 8 hours to about 48 hours.

In a preferred embodiment, the incubation temperature for multiplication of the species of *Brevibacillus* and subspecies of *Bacillus thuringiensis* according to the present invention is from 22 °C to 38 °C.

In a preferred embodiment, the species of *Brevibacillus* and subspecies of *Bacillus thuringiensis* are inoculated separately in the scaling process up to 180 L and mixed in the fermentors of 2.000 L as described for the present invention. For this purpose, in a preferred embodiment, after the cultivation of the *Brevibacillus* and subspecies of *Bacillus thuringiensis* in 1 L flasks of culture medium they are inoculated in two stainless steel flasks containing 1 L of culture medium each, and after the cultivation being inoculated in two 10 L flasks of culture medium, and then transferred to tanks containing 180 L of specific culture medium for each microorganism, whereby in Table 1 the specific culture medium for the species of *Brevibacillus* and subspecies of *Bacillus thuringiensis*; incubated for 24 to 168 hours. The air flow is preferably 1,0 to 15,0 Nm³/h (= 0,16 - 1,25 vvm).

In a preferred embodiment, the step of mixture of the species of *Brevibacillus* and subspecies of *Bacillus thuringiensis* is conducted at a temperature from 22 °C to 38 °C. The air flow is preferably 1,0 Nm³/h to 2,5 Nm³/h (= 0,0085 - 0,021 vvm). The pressure is preferably of 0,5 to 1,2 kgf/cm³. The agitation is preferably from 40 hz to 45 hz.

Preferably, the concentrations of microbiological actives comprise the range of 1,0×10⁷ - 1,0×10¹⁰ viable spores/mL for each species, and the essential oils employed in the agricultural composition, tea tree and citronella, are in the range between 0,01 - 5,0 % (v/v).

### EXAMPLES

### EXAMPLE 1 - SCALING OF THE MIXTURE

The species of *Brevibacillus* and subspecies of *Bacillus thuringiensis* are inoculated separately in flasks containing 100 mL of culture medium as described in Table 1, being incubated in orbital agitator of about 80-200 rpm, at 22-38 °C for approximately 8-48 hours. The next step in the scaling consists in the inoculation of stainless steel flasks containing 1 L of culture medium (Table 1) wherein the species are cultivated separately and incubated for approximately 8 - 48 hours, with air flow 0,25-1,0 Nm³/h (=4,16 - 16,67 vvm), at 22-38 °C.

After the incubation period, the cultivations of the species of *Brevibacillus* and subspecies of *Bacillus thuringiensis* are inoculated in stainless steel flasks containing 10 L of specific culture medium for each microorganism as described in table 1 and incubated for approximately 18 - 96 hours, with air flow 0,25 - 1,5 Nm³/h (= 0,41 - 2,5 vvm) and temperature varying from 22 to 38 °C.

**TABLE 1. CULTURE MEDIUM USED FOR THE GROWTH OF BREVIBACILLUS AND SUBSPECIES OF BACILLUS THURINGIENSIS UP TO THE SCALE OF 2.000 L.**

| | ***Reagents*** | *B*. *thuringiensis* | *B*. *laterosporus* |
|---|---|---|---|
| **01** | KH₂PO₄ | 0,5 - 5 g | - |
| **02** | CaCl₂.2H₂O | 0,1 - 1 g | - |
| **03** | MgSO₄.7H₂O | 0,1 - 1 g | - |
| **04** | MnCl₂ | 0,01 - 0,3 g | - |
| **05** | FeSO₄ | 0,01 - 0,3 g | - |
| **06** | ZnSO₄ | 0,01 - 0,3 g | - |
| **07** | Yeast extract | 5-50 g | 5-30 g |
| **08** | Glucose | 1-10 g | 1-10 g |
| **09** | Malt extract | - | 5-50 g |
| **10** | Sodium chloride | - | 2-10 g |
| **11** | Water | q.s. 1L | |

| | | | |
|---|---|---|---|
| q.s.: quantum sufficit. | | | |

After this period has lapsed, each culture containing two stainless steel flasks with 10 L of the culture medium are inoculated in a tank containing 180 L of the specific culture medium for each microorganism, and incubated for approximately 24 - 168 hours, with air flow 3,0- 10,0 Nm³/h (= 0,25 - 0,83 vvm) and temperature varying between 22 - 38 °C.

After the incubation in tanks of 180 to 300 L, they are then inoculated in three fermentors of 2.000 to 6.000 L containing the specific culture medium for each microorganism. Preferably, the process of sterilization of the culture medium is carried out for approximately 60 to 120 minutes, at a temperature of approximately 121 °C to approximately 130 °C. Preferably, the sterilization is carried out at a pressure of approximately 1,0 - 2,0 Kgf/cm². After the sterilization and cooling period, the tank containing the *Brevibacillus laterosporus, Bacillus thuringiensis* subsp. kurstaki and *Bacillus thuringiensis* subsp. aizawai are then inoculated to the fermentor of 2.000 to 6.000 L and the incubation time is preferably from 4 to 72 hours at a temperature of 22°C - 38°C. The air flow is preferably 1,0 Nm³/h a 2,5 Nm³/h (= 0,0085 - 0,021 vvm). The pressure is preferably 1,0 to 2,0 kgf/cm³. The agitation is preferably from 40 hz to 45 hz.

### EXAMPLE 2 - MIXTURE OF BREVIBACILLUS, BACILLUS THURINGIENSIS AND BOTANICAL EXTRACT IN BIOREACTOR.

For the mixture of the species of *Brevibacillus* and *Bacillus thuringiensis* in fermentor of 6.000 to 20.000 L, preferably the sterilization process of the fermentor is carried out for approximately 60 to 120 minutes, at a temperature of approximately 121 °C to approximately 130 °C. Preferably, the sterilization is carried out at a pressure of approximately 1,0 - 2,0 Kgf/cm². After the period of sterilization and cooling, the tank containing *Brevibacillus laterosporus, Bacillus thuringiensis* subsp. kurstaki and *Bacillus thuringiensis* subsp. aizawai and 0,01 - 5,00% of tea tree essential oil or citronella essential oil are then transferred to the fermentor of 6.000 to 20.000 L and the mixture time is preferably of 120 to 180 minutes at a temperature of 22°C - 38°C. The air flow is preferably 1,0 Nm³/h to 2,5 Nm³/h (= 0,0085 - 0,021 vvm). The pressure is preferably from 1,0 to 2,0 kgf/cm³. Agitation is preferably from 40 hz to 45hz.

Preferably, the product is filled in gallons, package in which the product is stored.

Alternatively, the scaling process of the cultures can be used for larger volumes, however, always respecting the proportion of each microorganism.

### EXAMPLE 3 - BREVIBACILLUS LATEROSPORORUS NEW SPECIES WITH BIOINSECTICIDE POTENTIAL AGAINST CATERPILLARS WITH AGRICULTURAL INTEREST.

Initially, there were carried out laboratory trials to validate the bioinsecticide effect in different concentrations of new species of *Brevibacillus lateresporus* against fall armyworm (*Spodoptera frugiperda*) inoculated in artificial diet (Figure 1). The concentration of 10⁷ UFC/mL of diet of the *Brevibacillus laterosporus* resulted in 100% mortality of the fall armyworm, similar result to the evaluated biological control, standard commercial market product containing *Bacillus thuringiensis* strain HD1, demonstrating the potential bioinsecticide effect of the bacteria.

### EXAMPLE 4 - BIOINSECTICIDE EFFECT OF THE AGRICULTURAL COMPOSITION WITH TWO SUBSPECIES OF BACILLUS THURINGIENESIS AND BREVIBACILLUS LATEROSPORUS POTENTIALIZING THE EFFECT WITH BOTANICAL EXTRACT IN THE COMPOSITION.

The development of an agricultural composition with two subspecies of *Bacillus thuringiensis* and a species of *Brevibacillus laterosporus* was evaluated in the wheat cultivation field searching for the best dosage against the fall armyworm (Figure 2), it was possible to find that the efficiency in the control with the dosage of 1L/ha of the biological was similar to the chemical control, and further, presented significant difference with efficiency 39% superior to the market reference biological control in the dosage 1 kg/ha. Surprisingly, the agricultural composition with addition of *B. laterosporus* presented a significantly superior control percentage to the treatment that employed only the subspecies of *B. thuringiensis,* disclosing great synergistic effect reached with the addition of B. *laterosporus.*

Moreover, it was possible to evaluate the potentializing effect of the botanical extract containing 0,01- 5% tea tree essential oil or 0,01-50 citronella essential oil against the fall armyworm (Figure 3). Although the percentage of mortality in the treatment with only the biological consortium was the same as the treatments with the consortium associated to the botanical extracts, tea tree and citronella, the velocity of the mortality was superior when evaluated by the Area Under the Mortality Progress Curve (AUMPC). In this manner, the adoption of the botanical extract containing the tea tree oil or citronella oil in the bioinsecticide agricultural composition becomes an important tool for the control of pests, thus potentializing the effect of the bioinsecticide action of the new species *Brevibacillus laterosporus* and two subspecies of *Bacillus thuringiensis* in a single product.

## Claims

1. Bioinsecticide agricultural composition, **characterized by** the fact that it comprises one or more species of *Brevibacillus laterosporus* and subspecies of *Bacillus thuringiensis,* preferably *Bacillus thuringiensis* subsp. *Kurstaki* and *Bacillus thuringiensis* subsp. *Aizawai,* associated with botanical extracts containing tea tree essential oil and/or citronella essential oil.

2. Bioinsecticide agricultural composition, according to claim 1, **characterized by** the fact that the concentration of microorganisms comprises the range from 1,0×10⁷ - 1,0×10¹⁰ viable spores/mL, and wherein the tea tree and citronella essential oils are in the range 0,01 - 5,0 % (v/v).

3. Bioinsecticide agricultural composition, according to claim 1, **characterized by** the fact that the use thereof is for application in, but not restricted to, soybean and corn, wherein the application thereof is preferably via foliar spraying, but not being restricted to the use, wherein said composition is used against insects of the Lepidoptera order.

4. Industrial induction process for obtaining bioinsecticide agricultural composition, as defined in claim 1, **characterized by** comprising the following steps:
(a) fermentation of the microorganisms *Bacillus thuringiensis* subsp. *kurstaki, Bacillus thuringiensis* subsp. *aizawai and Brevebacillus laterosporus,* not being restricted solely to the two genre cited, with the induction of endospores, as well as the formation of parasporal body and industrially induced crystals, wherein the industrial induction process for the formation of the parasporal body and crystals of interest is provided by different compositions of cultivation media and fermentation parameters (temperature, agitation, aeration and pressure, which are specific to each species of interest;
(b) next, after fermentation, there is carried out the mixture of microorganisms, and the mixture of the product wherein the botanical extracts are added at the end of the process of multiplication of the microorganisms, and the packaging of the formulated product.

5. Industrial induction process, according to claim 4, **characterized by** the fact that the industrial induction process of step (a):
occurs in fermentor environment for the species of *Brevibacillus* and subspecies of *Bacillus thuringienesis*;
occurs in fermentor environment of 2.000L to 6.000L for the species of *Brevibacillus* and subspecies of *Bacillus thuringienesis,* not being restricted solely to this volume;
is carried out at a temperature of approximately 22 °C to 38 °C;
for the species of *Brevibacillus* and subspecies of *Bacillus thuringienesis* is carried out at an air flow of approximately 3,0 Nm³/h (=0,25 vvm) to approximately 10,0 Nm³/h (=0,83 vvm); and
further comprises the sequential expansion of the culture of the species of *Brevibacillus* and subspecies of *Bacillus thuringienesis* for inoculation of the fermentation culture.

6. Industrial induction process, according to claim 4, **characterized by** the fact that the species of *Brevibacillus* and subspecies of *Bacillus thuringienesis* are inoculated separately.

7. Industrial induction process, according to claim 4, **characterized by** the fact that the sequential expansion is made in volumes of 100 mL, 1L, 10 L, 180 L and 2000 L.

8. Industrial induction process, according to claim 4, **characterized by** the fact that the species of *Brevibacillus* and subspecies of *Bacillus thuringienesis:*
are expanded by incubation in orbital agitator from 80 rpm to 200 rpm;
are expanded containing 100 mL of culture medium in glass flasks, 1 L and 10 L of culture medium in stainless steel flasks;
are expanded by incubation for 8 hours to 48 hours;
are incubated at an air flow of 0,25 Nm³/h to 1,5 Nm³/h (=0,41 - 2,5 vvm) for 1L and 10 L of the culture medium.

9. Industrial induction process, according to claim 8, **characterized by** the fact that after the segregated cultivation of the species in two stainless steel flasks of 10 L, said two flasks are inoculated in tanks containing 180 L of culture medium, wherein the strains are incubated for 24 to 168 hours.

10. Industrial induction process, according to claim 9, **characterized by** the fact that the species *Brevibacillus* and subspecies of *Bacillus thuringienesis* are incubated at an air flow of 1,0 to 15,0 Nm³/h (= 0,16 - 1,25 vvm) .

11. Industrial induction process, according to claim 4, **characterized by** the fact that the step of fermentation 2.000 L of the species of *Brevibacillus* and subspecies of *Bacillus thuringienesis* are carried out with pressure from 1,0 to 2,0 kgf/cm².

12. Industrial induction process, according to claim 4, **characterized by** the fact that the fermentation of the culture is by batch, and the fermentation step is carried out with agitation from 40 hz to 45 hz, with temperature from 22 °C to 38 °C, and air flow of 1,0 Nm³/h to 2,5 Nm³/h (= 0,0085 - 0,021 vvm).

13. Industrial induction process, according to claim 4, **characterized by** the fact that the mixture of the microorganisms in fermentor of 6.000L to 20.000L is carried out for 60 to 120 minutes, at a temperature of 121 °C to 130 °C.

14. Industrial induction process, according to claim 4, **characterized by** the fact that the process of mixture of the product in step (b) is carried out in fermentor of 6.000L to 20.000L, not being restricted solely to this volume, respecting the proportion of the mixture of different species of microorganisms, and the mixture time is preferably from 120 to 180 minutes at a temperature of 22°C - 38°C, the air flow is preferably 1,0 Nm³/h to 2,5 Nm³/h (= 0,0085 - 0,021 vvm), the pressure is preferably from 1,0 to 2,0 kgf/cm³, and the agitation is preferably from 40 hz to 45 hz.

15. Use of the bioinsecticide agricultural composition, as defined in claim 1, **characterized by** the fact that it is applied preferably via foliar spraying, but not being restricted to this use, and that it is for application in several agricultural cultures, such as corn, wheat, cotton, and for application in different dosages and number of applications.
